# EUROPÄISCHE PATENTANMELDUNG

(11) **EP 2 629 088 A1**
(43) Veröffentlichungstag der Anmeldung: **21.08.2013**
(21) Anmeldenummer: 13152467.0
(22) Anmeldetag: 24.01.2013
(51) Int. Cl.: G01N 29/036, B06B 1/08, G01R 33/18, G01N 29/24

(54) **Vorrichtung und Verfahren zum Bestimmen einer biochemischen Funktion eines Fluids**

(30) Priorität: 16.02.2012 DE 102012202336
(71) Anmelder: Siemens Aktiengesellschaft, 80333 München (DE)
(72) Erfinder: Hayden, Oliver, 91074 Herzogenaurach (DE); Rührig, Manfred, 91207 Lauf a.d. Pegnitz (DE)

(57) **Zusammenfassung**

Sensorvorrichtung (1) zur Bestimmung mindestens einer biochemischen Funktion eines Fluids (F) mit mindestens einem magnetoelastischen Kapillarrohr (2), durch welches das Fluid (F) geführt wird, wobei eine Resonanzfrequenz (fR) des magnetoelastischen Kapillarrohres (2), welche von einer Oberflächenbeladung der Innenwand des magnetoelastischen Kapillarrohres (2) durch das hindurchgeführte Fluid (F) abhängt, zur Bestimmung der biochemischen Funktion des Fluids (F) kontaktfrei auslesbar ist.

## Beschreibung

Die Erfindung betrifft eine Vorrichtung und ein Verfahren zur Bestimmung einer biochemischen Funktion eines Fluids, insbesondere eines Körperfluids.

In vielen Anwendungen ist es notwendig, biochemische Funktionen eines Fluids zu bestimmen. Ein Beispiel für eine derartige biochemische Funktion stellt die Thrombozytenfunktion von Blut dar, wobei Thrombozyten bei Herz- und Kreislauferkrankungen eine Rolle spielen. Dieselben Mechanismen, die bei einem Blutaustritt aus einem verletzten Gefäß dieses wieder verschließen, können für die Entstehung von thromboembolischen Gefäßverschlüssen verantwortlich sein. Es sind verschiedene Messmethoden zur Ermittlung einer Thrombozytenfunktion bekannt. Beispielsweise wird bei einem Platelet-Funktions-Analyzer gemäß dem PFA-100 System mit einer Messquelle eine In-vivo-Situation eines verletzten Blutgefäßes nachgebildet. Eine Membran, welche zusätzlich mit ADP oder Epinephrin beschichtet sein kann, enthält dabei eine winzige Öffnung, durch die das eingebrachte Blut mit hohen Scherkräften gesaugt wird. Die Thrombozyten haften dabei an der Membran und verschließen die Öffnung. Die Verschlußbildung wird durch eine Druckmessung charakterisiert. Die Zeitdauer in Minuten bis zum Verschluss der Membranöffnung durch den sich bildenden Thrombozytenpfropf dient dabei als Maß für die Thrombozytenfunktion. Das Gerinnungssystem eines Organismus ist hochkomplex und umfasst eine Vielzahl biochemischer Reaktionen. Ein Nachteil herkömmlicher Systeme, insbesondere auch des herkömmlichen Platelet-Funktions-Analyzers, besteht darin, dass ein Multiplexing durch parallele Messungen verschiedener biochemischer Reaktionen nicht oder nur mit erheblichem Aufwand möglich ist. Darüber hinaus ist es bei dem Platelet-Funktions-Analyzer notwendig, das entnommene Blut zu pipettieren. Dies ist naturgemäß relativ aufwendig und kann zudem nur durch geübte Personen durchgeführt werden.

Es ist daher eine Aufgabe der vorliegenden Erfindung, eine Vorrichtung und ein Verfahren zur Bestimmung mindestens einer biochemischen Funktion eines Fluids zu schaffen, welches die biochemischen Funktionen des Fluids sicher und mit geringem Aufwand ermitteln kann.

Diese Aufgabe wird erfindungsgemäß durch eine Sensorvorrichtung mit den in Patentanspruch 1 angegebenen Merkmalen gelöst.

Die Erfindung schafft demnach eine Sensorvorrichtung zur Bestimmung einer biochemischen Funktion eines Fluids mit mindestens einem magnetoelastischen Kapillarrohr, durch welches das Fluid geführt wird, wobei eine Resonanzfrequenz des magnetoelastischen Kapillarrohres, welche von einer Oberflächenbeladung der Innenwand des magnetoelastischen Kapillarrohres durch das hindurchgeführte Fluid abhängt, zur Bestimmung der biochemischen Funktion des Fluids kontaktfrei auslesbar ist.

Bei einer möglichen Ausführungsform der erfindungsgemäßen Sensorvorrichtung weist diese eine Sendespule auf, welche das magnetoelastische Kapillarrohr kontaktfrei zu mechanischen Schwingungen anregt.

Bei einer weiteren möglichen Ausführungsform der erfindungsgemäßen Sensorvorrichtung weist diese zusätzlich eine Aufnehmerspule auf, welche ein von dem magnetoelastischen Kapillarrohr erzeugtes Magnetfeld zur Ermittlung von dessen Resonanzfrequenz erfasst.

Bei einer möglichen Ausführungsform der erfindungsgemäßen Sensorvorrichtung sind die Sendespule und die Aufnehmerspule um das magnetoelastische Kapillarrohr herumgewickelt.

Bei einer möglichen Ausführungsform ist dabei die Sendespule um das magnetoelastische Kapillarrohr herumgewickelt und die Aufnehmerspule ihrerseits um die Sendespule herumgewickelt.

Bei einer weiteren möglichen Ausführungsform der erfindungsgemäßen Sensorvorrichtung sind mehrere magnetoelastische Kapillarrohre vorgesehen, die jeweils ein unterschiedliches Elastizitätsmodul aufweisen.

Bei einer weiteren möglichen Ausführungsform der erfindungsgemäßen Sensorvorrichtung weisen die magnetoelastischen Kapillarrohre eine unterschiedliche Länge auf.

Bei einer weiteren möglichen Ausführungsform der erfindungsgemäßen Sensorvorrichtung weisen die magnetoelastischen Kapillarrohre einen unterschiedlichen Durchmesser auf.

Bei einer weiteren möglichen Ausführungsform der erfindungsgemäßen Sensorvorrichtung haben die magnetoelastischen Kapillarrohre eine unterschiedliche Oberflächenbeschichtung, welche unterschiedliche Zugabereagenzien zur Ermittlung verschiedener biochemischer Funktionen des Fluids aufweisen.

Bei einer möglichen Ausführungsform der erfindungsgemäßen Sensorvorrichtung besteht das magnetoelastische Kapillarrohr aus einem transparenten Material, beispielsweise aus Glas oder einem transparenten Kunststoff.

Bei einer möglichen Ausführungsform der erfindungsgemäßen Sensorvorrichtung ist das Elastizitätsmodul des magnetoelastischen Kapillarrohres über eine Vormagnetisierung einstellbar.

Bei einer weiteren möglichen Ausführungsform der erfindungsgemäßen Sensorvorrichtung besteht das magnetoelastische Kapillarrohr aus einem Material mit hoher Magnetostriktionskonstante.

Bei einer möglichen Ausführungsform der erfindungsgemäßen Sensorvorrichtung besteht das magnetoelastische Kapillarrohr aus hochmagnetorestriktiven CoFe-Legierungen.

Bei einer alternativen Ausführungsform der erfindungsgemäßen Sensorvorrichtung besteht das magnetoelastische Kapillarrohr aus hochmagnetorestriktiven Seltenerd-Eisen-Legierungen.

Bei einer weiteren möglichen Ausführungsform besteht das magnetoelastische Kapillarrohr der Sensorvorrichtung aus magnetorestriktiven amorphen Legierungen.

Bei einer weiteren möglichen Ausführungsform der erfindungsgemäßen Sensorvorrichtung wird als biochemische Funktion durch die Sensorvorrichtung eine Thrombozytenfunktion von Blut bestimmt.

Die Erfindung schafft ferner ein Blutentnahmeröhrchen mit einer darin integrierten Sensorvorrichtung zur Bestimmung mindestens einer biochemischen Funktion eines Fluids, wobei die in dem Blutentnahmeröhrchen integrierte Sensorvorrichtung mindestens ein magnetoelastisches Kapillarrohr aufweist, durch welches das Fluid geführt wird, wobei eine Resonanzfrequenz des magnetoelastischen Kapillarrohres, welche von einer Oberflächenbeladung der Innenwand des magnetoelastischen Kapillarrohres durch das hindurchgeführte Fluid abhängt, zur Bestimmung der biochemischen Funktion des Fluids kontaktfrei auslesbar ist.

Bei einer möglichen Ausführungsform des erfindungsgemäßen Blutentnahmeröhrchens ist an dem Blutentnahmeröhrchen eine Nadel angebracht, durch welche venöses Blut aus einem Organismus entnehmbar ist und in eine erste Blutaufnahmekammer des Blutentnahmeröhrchens gelangt.

Bei einer weiteren möglichen Ausführungsform des erfindungsgemäßen Blutentnahmeröhrchens mit integrierter Sensorvorrichtung ist die integrierte Sensorvorrichtung von der ersten Blutaufnahmekammer durch eine erste Berstscheibe oder ein Ventil getrennt.

Bei einer weiteren möglichen Ausführungsform des erfindungsgemäßen Blutentnahmeröhrchens gelangt das durch die magnetoelastischen Kapillarrohre der Sensorvorrichtung hindurchgeführte Blut in eine zweite Blutaufnahmekammer des Blutentnahmeröhrchens, die mit einem Unterdruck beaufschlagbar ist.

Bei einer weiteren möglichen Ausführungsform des erfindungsgemäßen Blutentnahmeröhrchens ist zwischen der Sensorvorrichtung und der zweiten Blutaufnahmekammer eine zweite Berstscheibe oder Ventil vorgesehen.

Die Erfindung schafft ferner ein Verfahren mit den in Patentanspruch 16 angegebenen Merkmalen.

Die Erfindung schafft demnach ein Verfahren zum Bestimmen mindestens einer biochemischen Funktion eines Fluids, wobei eine Resonanzfrequenz eines magnetoelastischen Kapillarrohres, durch welches das Fluid geführt wird, zur Bestimmung der biochemischen Funktion des Fluids kontaktfrei ausgelesen wird,
wobei die Resonanzfrequenz des magnetoelastischen Kapillarrohres von einer Oberflächenbeladung der Innenwandung des magnetoelastischen Kapillarrohres durch das hindurchgeführte Fluid abhängt.

Im Weiteren werden mögliche Ausführungsformen des erfindungsgemäßen Verfahrens und der erfindungsgemäßen Vorrichtung zur Bestimmung einer biochemischen Funktion eines Fluids unter Bezugnahme auf die beigefügten Figuren näher beschrieben.

Es zeigen:
- Fig. 1: ein Diagramm zur Darstellung der prinzipiellen Funktionsweise des erfindungsgemäßen Verfahrens und der erfindungsgemäßen Vorrichtung zur Bestimmung einer biochemischen Funktion eines Fluids;
- Fig. 2: ein Blockschaltbild zur Darstellung eines Ausführungsbeispiels der erfindungsgemäßen Sensorvorrichtung zur Bestimmung einer biochemischen Funktion eines Fluids.

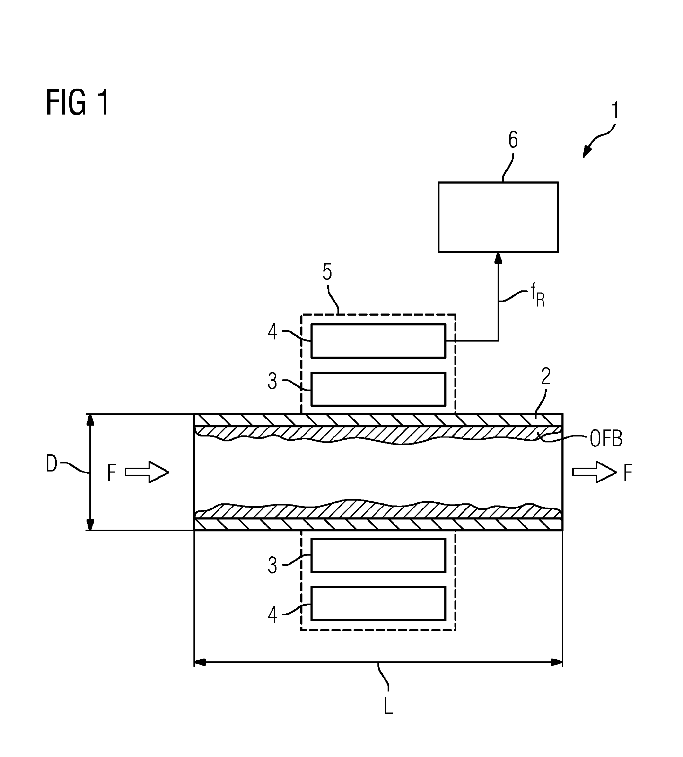

Wie man aus Fig. 1 erkennen kann, wird bei der erfindungsgemäßen Sensorvorrichtung 1 ein Fluid F durch ein magnetoelastisches Kapillarrohr 2 geführt. Bei dem Fluid F kann es sich um eine Körperflüssigkeit, beispielsweise Urin oder venöses Blut, handeln. Das magnetoelastische Kapillarrohr 2 bildet einen Biegeschwinger dessen mechanische Resonanzfrequenz von der Länge L des magnetoelastischen Kapillarrohres 2 sowie einem Durchmesser D des Kapillarrohres 2 abhängt. Außerdem geht in die Resonanzfrequenz noch die elastischen Eigenschaften in Form des Elastizitätsmoduls (Zugmodul, Elastizitätskoeffizient oder Youngscher Modul) des Materials ein. Der Elastizitäts- oder E-Modul hängt von der Umgebung des Kapillarrohrs ab, insbesondere von der Oberflächenbeladung OFB der Innenwand des Kapillarrohres 2 durch die Substanzen, die an der Innenwandung des Kapillarrohres 2 haften bzw. sich dort anlagern. Weiterhin kann der E-Modul E des Kapillarrohres 2 bzw. des Biegeschwingers von der Viskosität des hindurchströmenden Fluids F abhängen. Darüber hinaus hängt der Elastizitätsmodul E des Kapillarrohres, über die magnetostriktiven Eigenschaften des Materials, aus dem das Kapillarrohr 2 hergestellt ist, vom Magnetisierungszustand des Kapillarrohres 2 ab. So kann z.B. der E-Modul des magnetoelastischen Kapillarrohres 2 über eine Vormagnetisierung des magnetorestriktiven Materials eingestellt werden. Bei einer möglichen Ausführungsform der erfindungsgemäßen Sensorvorrichtung 1 besteht das magnetoelastische Kapillarrohr 2 aus einem Material mit einer hohen Magnetostriktionskonstante. Beispielsweise besteht das magnetoelastische Kapillarrohr 2 aus einer hochmagnetorestriktiven CoFe-Legierung. Bei einer weiteren möglichen Ausführungsform kann das magnetoelastische Kapillarrohr 2 eine hochmagnetostriktive Seltenerd-Eisen-Legierung aufweisen (z.B. Terfenol® (= TbDyFe)). Bei einer weiteren möglichen Ausführungsform versteht das magnetoelastische Kapillarrohr 2 aus einer hochmagnetorestriktiven Fe-Ga Legierung (Galfenol®). Eine Materialklasse, die für die Ausbildung des Kapillarrohres 2 besonders gut geeignet ist, sind hochmagnetorestriktive amorphe CoFe-Legierungen, die durch Rascherstarrung aus der Schmelze in den amorphen Zustand abgeschreckt werden. Der magnetomechanische Kopplungsfaktor d.h. die Kopplung zwischen mechanischen Schwingungen eines Materials und den über die Magnetostriktion daran gekoppelten Magnetisierungsänderungen des Materials ist gerade in amorphen (glasartigen) Materialien wegen des großen E-Moduls (mechanische Härte) bei gleichzeitig hoher Magnetostriktion und leichten Magnetisierbarkeit (magnetische Weichheit) besonders hoch.

Der Querschnitt des Kapillarrohres 2 kann bei einer möglichen Ausführungsform rund sein. Bei alternativen Ausführungsformen können auch Kapillarrohre mit anderen Querschnitten, beispielsweise rechteckigen Querschnitten, eingesetzt werden.

Wie man in Fig. 1 erkennen kann, ist um das magnetoelastische Kapillarrohr 2 eine Magnetisierungsspule 3 gewickelt, welche das magnetoelastische Kapillarrohr 2 kontaktfrei mit einem magnetischen Wechselfeld der Frequenz f beaufschlagt. Durch das Wechselfeld vermittelt über die Magnetostriktion des Kapillarmaterials wird das Kapillarrohr 2 zu mechanischen Schwingungen anregt. Um die Magnetisierungsspule 3 herum ist eine Aufnehmerspule 4 gewickelt, welche die durch die mechanischen Schwingungen in dem magnetoelastischen Kapillarrohr 2 erzeugten Magnetisierungsänderungen induktiv erfasst. Stimmt man die Frequenz f des Erregerfeldes über einen bestimmten Frequenzbereich fₘᵢₙ<f_{R}<fₘₐₓ durch, so kann man die Resonanzfrequenz f_{R} des Kapillarrohres 2 bestimmen, bei der die Schwingungsamplitude maximal wird. Bei der in Fig. 1 dargestellten Ausführungsform ist die Aufnehmerspule 4 um die Magnetisierungsspule 3 herumgewickelt. Bei einer weiteren möglichen Ausführungsform können die Magnetisierungsspule 3 und die Aufnehmerspule 4 entlang des Kapillarrohres 2 versetzt vorgesehen sein. Die Magnetisierungsspule 3 und die Aufnehmerspule 4 bilden zusammen einen Signalabnehmer 5, welcher die Resonanzfrequenz f_{R} des Kapillarrohres 2 an eine Auswerteeinheit 6 überträgt. Bildet sich an der Innenwandung des Kapillarrohres 2 eine Oberflächenbeladung OFB, wie in Fig. 1 angedeutet, verändert sich das Elastizitätsmodul E des Kapillarrohres 2 und somit dessen Resonanzfrequenz f_{R}. Die Oberflächenbeladung OFB kann durch eine biochemische Reaktion des hindurchgeführten Fluids F hervorgerufen werden. Das Kapillarrohr 2 bzw. der Biegebalken bzw. Biegeschwinger wird über ein Magnetfeld mittels der Magnetisierungsspule 3 kontaktfrei zu Schwingungen angeregt. Die angeregten Schwingungen des magnetoelastischen Kapillarrohres 2 klingen nach Pulsende in charakteristischer Art und Weise ab und erzeugen dabei ihrerseits Magnetfelder (Streufelder), die von der Aufnehmerspule 4 detektiert werden können. Durch Frequenzmodulation ist es möglich, die Resonanzfrequenzen f_{R} aller in dem Abfragefeld angeordneten Kapillaren bzw. magnetoelastischen Kapillarrohre 2 auszulesen.

Bei einer weiteren möglichen Ausführungsform wird der Biegeschwinger über ein Magnetfeld mittels der Magnetisierungsspule 3 kontaktfrei zu Schwingungen angeregt. Die Magnetisierungsänderungen in der Kapillare werden von einer Aufnehmerspule 4 detektiert, die dabei so ausgestaltet ist, dass die Felder der Magnetisierungsspule 3 die Aufnehmerspule nicht durchdringen oder sich kompensieren. Eine Nichtdurchdringung kann dabei realisiert werden, indem die Ebenen der beiden Spulen 3,4 näherungsweise senkrecht stehen. Eine Kompensation lässt sich realisieren, indem die Aufnehmerspule 4 aus zwei näherungsweise identischen Teilspulen besteht, die in Reihe geschaltet sind und deren Wicklung gegensinnig ausgeführt ist. Zweckmäßigerweise durchdringt das Kapillarrohr 2 dann lediglich eine der beiden Teilspulen, während die andere Teilspule nur den Luftfluss einschleißt.

Dies führt dazu, dass sich die Magnetflussänderungen, die durch die Magnetisierungsspule 3 verursacht werden, in den beiden Teilenspulen der so ausgeführten Aufnehmerspule 4 gerade aufheben, während die Magnetisierungsänderung in dem Kapillarrohr 2, das nur eine der beiden Teilspulen durchdringt, erfasst werden.

Bei einer möglichen Ausführungsform kann als biochemische Funktion durch die Sensorvorrichtung 1 eine Thrombozytenfunktion von hindurchfließendem Blut ermittelt werden. Bei einer möglichen Ausführungsform kann das Kapillarrohr (z.B. mit Kollagen) zur Aktivierung der Thrombozyten beschichtet sein. Alternativ kann ein lösliche Aktivator (z.B. ADP) der Blutprobe zugesetzt werden. Als magnetoelastische Schwinger bzw. magnetoelastische Kapillarrohre 2 werden bevorzugt Kapillarrohre, die aus einem Material mit hoher Magnetostriktionskonstante bestehen und gleichzeitig ein großes Elastizitätsmodul E aufweisen eingesetzt, da diese einen hohen magnetomechanischen Kopplungsfaktor aufweisen. Dadurch ergibt sich eine hohe Schwingungsamplitude mit relativ großer Zeitkonstante im Abklingsignal. Das Auslesen der Resonanzfrequenz f_{R} erlaubt es, die biochemische Funktion des hindurchströmenden Fluids F, beispielsweise Blut, kontaktfrei zu bestimmen. Die Strömungsgeschwindigkeit des durch das Kapillarrohre 2 hindurchströmenden Fluids F hängt von dem Druckgefälle ΔP zwischen den beiden Enden des magnetoelastischen Kapillarrohres 2 ab, wobei dieses Druckgefälle bei einer möglichen Ausführungsform des erfindungsgemäßen Verfahrens einstellbar sein kann. Darüber hinaus ist es möglich, dass das zu analysierende Fluid F, beispielsweise Blut, durch eine vor dem magnetoelastischen Kapillarrohr 2 angeordneten Inkubationseinrichtung mit Reagenzien beaufschlagt wird, die mit dem Fluid F eine biochemische Reaktion eingehen, wodurch beispielsweise die in Fig. 1 dargestellte Oberflächenbeladung OFB erzeugt wird, die ihrerseits die Resonanzfrequenz f_{R} des magnetoelastischen Kapillarrohres 2 verändert.

Bei einer möglichen Ausführungsform können als Sensoreinheit auch mehrere magnetoelastische Kapillarrohre 2 mit unterschiedlicher Länge L, Durchmesser D und Oberflächenbeschichtung, beispielsweise Aktivatoren zur Blutgerinnung, verwendet werden. Bei einer möglichen Ausführungsform weisen die Kapillarrohre 2 Resonanzfrequenzen f_{R} im Kilohertz-Bereich auf. Weiterhin ist es möglich, dass mehrere Kapillarrohre bzw. ein Array von Kapillarrohren 2 in einem Blutentnahmeröhrchen integriert ist.

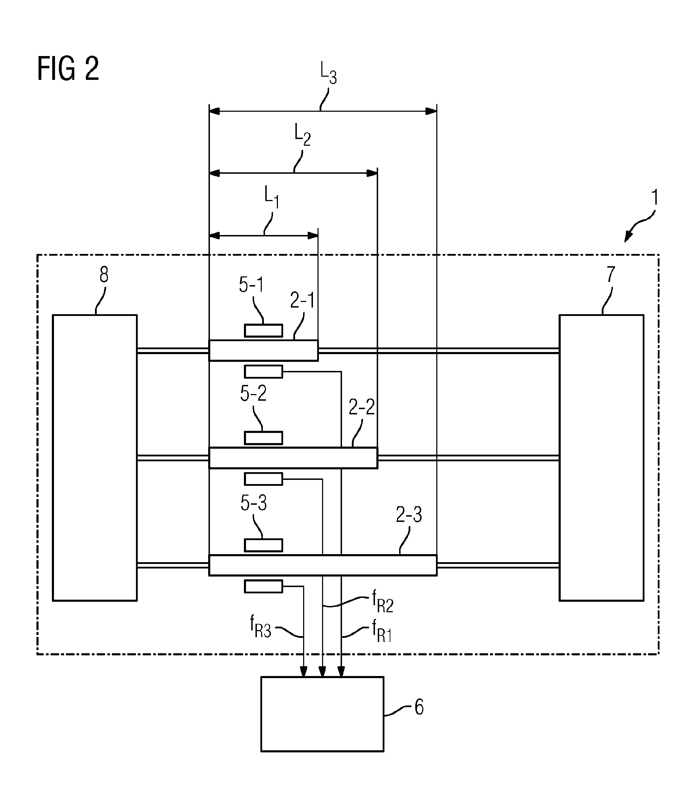

Fig. 2 zeigt ein Ausführungsbeispiel, bei dem die Sensorvorrichtung 1 mehrere magnetoelastische Kapillarrohre 2-1, 2-2, 2-3 aufweist, die zwischen der ersten Fluidaufnahmekammer 7 und einer zweiten Fluidaufnahmekammer 8 angeordnet sind. Bei dem in Fig. 2 dargestellten Ausführungsbeispiel weisen die unterschiedlichen Kapillarrohre unterschiedliche Längen L1, L2, L3 und somit unterschiedliche Elastizitätsmodule E auf. Auch die Durchmesser D und die Materialien der verschiedenen Kapillarrohre 2 können unterschiedlich sein. Die in Fig. 2 dargestellte Sensorvorrichtung 1 kann in einem Blutentnahmeröhrchen integriert sein, wobei der ersten Fluidaufnahmekammer 7 ein Körperfluid, beispielsweise Fluid, über eine Nadel zugeführt wird. Durch Anlegen eines entsprechenden Druckgefälles zwischen der ersten Fluidaufnahmekammer 7 und der zweiten Fluidaufnahmekammer 8 wird erreicht, dass Fluid von der ersten Fluidaufnahmekammer 7 durch die parallel angeordneten Kapillarrohre 2-1, 2-2, 2-3 hin zu der zweiten Fluidaufnahmekammer 8 strömt. Bei einer möglichen Ausführungsform kann an die zweite Fluidaufnahmekammer 8 ein Unterdruck angelegt werden, sodass ein Druckgefälle zwischen der ersten und zweiten Fluidaufnahmekammer entsteht. Die von den verschiedenen Signalabnehmern 5-i ermittelten Resonanzfrequenzen fRᵢ können, wie in Fig. 2 dargestellt, zur weiteren Auswertung einer externen Auswerteeinheit 6 zugeführt werden. Bei einer möglichen Ausführungsform kann die Auswerteeinheit 6 zur Auswertung der gemessenen Resonanzfrequenzen fRᵢ ebenfalls in dem Blutentnahmeröhrchen integriert sein. An dem Blutentnahmeröhrchen kann eine Nadel angebracht werden, durch welche venöses Blut aus einem Organismus entnehmbar ist und in die erste Blutaufnahmekammer 7 des Blutentnahmeröhrchens gelangt. Bei einer möglichen Ausführungsform ist die integrierte Sensorvorrichtung, d.h. die parallel angeordneten Kapillarrohre 2-i, durch Berstscheiben von der ersten Blutaufnahmekammer 7 getrennt. Die zweite Blutaufnahmekammer 8 bildet ein Waste Compartment, in dem das durch die Kapillarrohre 2-i hindurchgeführte Blut aufgenommen wird, ohne dass es nach außen gelangt. Zwischen der zweiten Blutaufnahmekammer 8 und der integrierten Sensorvorrichtung können ebenfalls Berstscheiben vorgesehen sein, die bei Anlegen eines entsprechenden Unterdruckes bersten, sodass das Fluid F durch die Kapillarrohre 2-i strömen kann. Wie man aus Fig. 2 erkennen kann, können durch die parallele Anordnung der verschiedenen magnetoelastischen Kapillarrohre 2-i, die jeweils über unterschiedliche Elastizitätsmodule, aber auch unterschiedliche Inkubationseinrichtungen bzw. Beschichtungen verfügen, gleichzeitig mehrere biochemische Funktionen des Fluids F erfasst werden. Ein Sensor-Array bzw. ein Sensorfeld mit mehreren parallel angeordneten Kapillarrohren 2-i kann in einfacher Weise platzsparend in einem Gerät integriert werden, insbesondere in einem Blutentnahmeröhrchen. Die erfassten Resonanzfrequenzen f_{Ri} können über eine Datenschnittstelle drahtlos oder drahtgebunden an die externe Auswerteeinheit 6 übermittelt werden. Die erfindungsgemäße Sensorvorrichtung 1 kann auch in einer austauschbaren Kassette oder dergleichen integriert sein. Durch Veränderung bzw. Variation des Durchmessers D der verschiedenen Kapillarrohre 2 kann zudem das Fluid F mit unterschiedlichen Scherkräften beaufschlagt werden, die beispielsweise eine Rolle bei der Messung der Blutgerinnung spielen können. Aufgrund der Vielzahl der parallel integrierten Kapillarrohre 2-i bietet die erfindungsgemäße Sensorvorrichtung 1 gewissermaßen eine Multiplexfunktion zur gleichzeitigen bzw. parallelen Messung einer Vielzahl von unterschiedlichen biochemischen Funktionen des zu untersuchenden Fluids F. Dabei sind die verschiedenen Kapillarrohre 2 einfach und mit geringem Platzbedarf in einem Gerät integrierbar. Die Beschichtung der unterschiedlichen Kapillarrohre 2 mit unterschiedlichen Reagenzien erlaubt es, unterschiedlichste biochemische Funktionen des hindurchströmenden Fluids F zu erfassen. Weiterhin erlaubt die Variation des Durchmessers D eine Beaufschlagung des zu untersuchenden Fluids F mit unterschiedlichen Scherkräften. Die Sende- und Aufnehmerspule können in einfacher Weise platzsparend um die Kapillarrohre 2-i herumgewickelt werden, wodurch die Integration in einem Gerät, beispielsweise einem Blutentnahmeröhrchen, zusätzlich erleichtert wird. Das untersuchte Fluid F kann nach erfolgter Untersuchung für weitere Nachmessungen in einfacher Weise in einem Blutentnahmeröhrchen, in welchem die erfindungsgemäße Sensorvorrichtung 1 integriert ist, gelagert werden.

## Patentansprüche

1. Sensorvorrichtung (1) zur Bestimmung mindestens einer biochemischen Funktion eines Fluids (F) mit mindestens einem magnetoelastischen Kapillarrohr (2), durch welches das Fluid (F) geführt wird,
wobei eine Resonanzfrequenz (fR) des magnetoelastischen Kapillarrohres (2), welche von einer Oberflächenbeladung der Innenwand des magnetoelastischen Kapillarrohres (2) durch das hindurchgeführte Fluid (F) abhängt, zur Bestimmung der biochemischen Funktion des Fluids (F) kontaktfrei auslesbar ist.

2. Sensorvorrichtung nach Anspruch 1,
wobei eine Magnetisierungsspule (3), welche das magnetoelastische Kapillarrohr (2) kontaktfrei zu mechanischen Schwingungen anregt, und
eine Aufnehmerspule (4)vorgesehen ist, welche die in dem magnetoelastischen Kapillarrohr (2) erzeugten Magnetisierungsänderungen induktiv erfasst.

3. Sensorvorrichtung nach Anspruch 1 oder 2,
wobei die Magnetisierungsspule (3) und die Aufnehmerspule (4) konzentrisch gewickelt sind und das Kapillarrohr (2) die von beiden Spulen (3,4) begrenzten Flächen durchdringt.

4. Sensorvorrichtung nach Anspruch 1 oder 2, wobei die Spulenflächen der Magnetisierungsspule (3) und der Aufnehmerspule (4) einen Winkel einschließen.

5. Sensorvorrichtung nach nach einem der vorangehenden Ansprüche, wobei die Aufnehmerspule (4) derart ausgestaltet oder orientiert ist, dass das sie durchdringende Magnetfeld minimal ist.

6. Sensorvorrichtung 1 bis 5,
wobei mehrere magnetoelastische Kapillarrohre (2) vorgesehen sind, die jeweils ein unterschiedliches Elastizitätsmodul aufweisen.

7. Sensorvorrichtung nach Anspruch 6,
wobei die magnetoelastischen Kapillarrohre (2) eine unterschiedliche Länge (L) und/oder einen unterschiedlichen Durchmesser (D) aufweisen.

8. Sensorvorrichtung nach Anspruch 6 oder 7,
wobei die magnetoelastischen Kapillarrohre (2) eine unterschiedliche Oberflächenbeschichtung haben, welche unterschiedliche Zugabereagenzien zur Ermittlung verschiedener biochemischer Funktionen des Fluids (F) aufweisen.

9. Sensorvorrichtung nach Anspruch 6, wobei das Elastizitätsmodul des magnetoelastischen Kapillarrohres (2) über eine Vormagnetisierung einstellbar ist.

10. Sensorvorrichtung nach Anspruch 9,
wobei das magnetoelastische Kapillarrohr (2) aus einem Material mit hoher Magnetostriktionskonstante besteht, insbesondere hochmagnetorestriktive CoFe-Legierungen hochmagnetostriktiven amorphen Co Fe-Legierungen, hochmagnetostriktiven Seltenerd-Eisen-Legierungen oder hochmagnetorestriktive Ga-Fe-Legierungen.

11. Sensorvorrichtung nach einem der vorangehenden Ansprüche 1 bis 10,
wobei als biochemische Funktion durch die Sensorvorrichtung (1) eine Thrombozytenfunktion von Blut bestimmt wird.

12. Blutentnahmeröhrchen mit einer darin integrierten Sensorvorrichtung (1) nach einem der vorangehenden Ansprüche 1 bis 11.

13. Blutentnahmeröhrchen nach Anspruch 12,
wobei an dem Blutentnahmeröhrchen eine Nadel angebracht ist, durch welche venöses Blut aus einem Organismus entnehmbar ist und in eine erste Blutaufnahmekammer (7) des Blutentnahmeröhrchens gelangt.

14. Blutentnahmeröhrchen nach Anspruch 12 oder 13,
wobei die integrierte Sensorvorrichtung (1) von der ersten Blutaufnahmekammer (7) durch eine erste Berstscheibe oder ein Ventil getrennt ist.

15. Blutentnahmeröhrchen nach einem der vorangehenden Ansprüche 12 bis 14,
wobei das durch die magnetoelastischen Kapillarrohre (2) der Sensorvorrichtung (1) hindurchgeführte Blut in eine zweite Blutaufnahmekammer (8) des Blutentnahmeröhrchens gelangt, die mit einem Unterdruck beaufschlagbar ist.

16. Blutentnahmeröhrchen nach Anspruch 15,
wobei zwischen der Sensorvorrichtung (1) und der zweiten Blutaufnahmekammer (8) eine zweite Berstscheibe oder ein Ventil vorgesehen ist.

17. Verfahren zum Bestimmen mindestens einer biochemischen Funktion eines Fluids,
wobei eine Resonanzfrequenz (fR) eines magnetoelastischen Kapillarrohres (2), durch welches das Fluid (F) geführt wird, zur Bestimmung der biochemischen Funktion des Fluids (F) kontaktfrei ausgelesen wird,
wobei die Resonanzfrequenz (fR) des magnetoelastischen Kapillarrohres (2) von einer Oberflächenbeladung der Innenwandung des magnetoelastischen Kapillarrohres (2) durch das hindurchgeführte Fluid (F) abhängt.
